# EUROPEAN PATENT APPLICATION

(11) **EP 2 071 008 A1**
(43) Date of publication of application: **17.06.2009**
(21) Application number: 07122223.6
(22) Date of filing: 04.12.2007
(51) Int. Cl.: C10M 133/46, C10M 135/32, C10M 141/08, C07D 233/04, C07D 233/48, C07D 233/72, C10M 133/44, C10M 135/16

(54) **Lubricating composition comprising an imidazolidinethione and an imidazolidone**

(71) Applicant: SHELL INTERNATIONALE RESEARCH MAATSCHAPPIJ B.V., 2596 HR Den Haag (NL)
(72) Inventor: The designation of the inventor has not yet been filed
(74) Representative: Cramwinckel, Michiel

(57) **Abstract**

The present invention provides a lubricating composition comprising:
- a base oil; and
- one or more imidazolid(inethi)one compounds having formula (I):

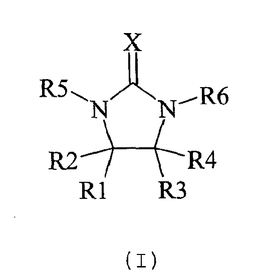

wherein X is O or S;
R¹, R², R³ and R⁴ are independently hydrogen, alkyl, alkenyl, aryl, aralkyl or NYR⁷; R⁷ is alkyl, alkenyl, aralkyl or R⁷ groups combine with N atoms to which they are attached and C atoms of an imidazolidinethione ring to form a five- to seven-membered heterocyclic ring; Y is hydrogen, alkyl, alkenyl, aryl, aralkyl, -CHR⁸-CHR⁹-COOR¹⁰, -CR¹¹R¹²NHR¹³ or -C(Z)NHR¹⁴ or oxyl ; R⁸ and R⁹ are independently hydrogen or C₁-C₄ alkyl; R¹⁰, R¹¹ and R¹² are independently hydrogen, alkyl, alkenyl, aryl or aralkyl; R¹³ and R¹⁴ are independently alkyl, alkenyl, aralkyl or aryl; Z is O or S; provided that when X is S, one of R¹ and R² and one of R³ and R⁴ are not at the same time NYR⁷;
or R¹ and R³ combine with carbon atoms of an imidazolidinethione ring to form a C₅-C₈ cycloalkyl or cycloalkenyl ring;
or R² and R⁵ combine with carbon atoms of an imidazolidinethione ring to form a C₅-C₈ cycloalkyl or cycloalkenyl ring;
or R¹ and R² combine to form a double bond with an O or S atom;
R⁵ and R⁶ are independently hydrogen, alkyl, alkenyl, aryl, aralkyl, -CHR⁸-CHR⁹-COOR¹⁰, -CR¹¹R¹²NHR¹³ or -C(Z)NHR¹⁴.

## Description

The present invention relates to a lubricating composition for particular use in internal combustion engines.

In practice various lubricating compositions are known. Zinc dialkyldithiophosphates (ZDDP) are widely used as lubricant additives in lubricating compositions for improving the anti-wear properties. Two important disadvantages of these ZDDP compounds are that an ash residue is produced by the zinc when the additive is consumed, and that phosphorus is known to affect the efficiency of catalytic converters in motor vehicles, thereby causing emissions problems.

In the field several non-metallic, non-phosphorus-containing oil-soluble additives for lubricating compositions have been suggested. As an example, EP 1 394 243 A1 discloses a composition comprising (a) from 0.1 to 20% of at least one specific amino-subsituted imidazolidinethione compound; and (b) a lubricating oil.

It is an object of the present invention to provide alternative lubricating compositions.

It is another object to provide a lubricating composition with improved friction reduction properties.

One or more of the above or other objects are obtained by the present invention by providing a lubricating composition comprising:
- a base oil; and
- one or more imidazolid(inethi)one compounds having formula (I): 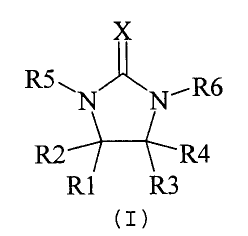 wherein X is O or S;
   R¹, R², R³ and R⁴ are independently hydrogen, alkyl, alkenyl, aryl, aralkyl or NYR⁷; R⁷ is alkyl, alkenyl, aralkyl or R⁷ groups combine with N atoms to which they are attached and C atoms of an imidazolidinethione ring to form a five- to seven-membered heterocyclic ring; Y is hydrogen, alkyl, alkenyl, aryl, aralkyl, -CHR⁸-CHR⁹-COOR¹⁰, -CR¹¹R¹²NHR¹³ or -C(Z)NHR¹⁴ or oxyl; R⁸ and R⁹ are independently hydrogen or C₁-C₄ alkyl; R¹⁰, R¹¹ and R¹² are independently hydrogen, alkyl, alkenyl, aryl or aralkyl; R¹³ and R¹⁴ are independently alkyl, alkenyl, aralkyl or aryl; Z is O or S; provided that when X is S, one of R¹ and R² and one of R³ and R⁴ are not at the same time NYR⁷;
   or R¹ and R³ combine with carbon atoms of an imidazolidinethione ring to form a C₅-C₈ cycloalkyl or cycloalkenyl ring;
   or R² and R⁵ combine with carbon atoms of an imidazolidinethione ring to form a C₅-C₈ cycloalkyl or cycloalkenyl ring;
   or R¹ and R² combine to form a double bond with an O or S atom;
   R⁵ and R⁶ are independently hydrogen, alkyl, alkenyl, aryl, aralkyl, -CHR⁸-CHR⁹-COOR¹⁰, -CR¹¹R¹²NHR¹³ or -C(Z)NHR¹⁴.

Surprisingly, the lubricating compositions according to the present invention exhibit - apart form desired anti-wear and anti-rust properties - improved friction reduction properties.

For the purpose of the present invention, an "alkyl" group is a saturated hydrocarbyl group having from 1 to 22 carbon atoms in a linear, branched or cyclic arrangement, and having from 0 to 2 oxygen, nitrogen or sulfur atoms. Substitution on alkyl groups of one or more halo, hydroxy, alkoxy, alkanoyl or amido groups is permitted; alkoxy, alkanoyl and amido groups may in turn be substituted by one or more halo substituents. In one preferred embodiment, alkyl groups contain from 1 to 12 carbon atoms and from 0 to 1 oxygen, nitrogen or sulfur atoms; in another preferred embodiment, alkyl groups contain from 4 to 22 carbon atoms, and more preferably, no heteroatoms.

An "alkenyl" group is an "alkyl" group in which at least one single bond has been replaced with a double bond. A "difunctional alkyl" or "difunctional alkenyl" group is an alkyl or alkenyl group having two points of attachment on the same or different carbon atoms, e.g., - CH₂-, -CH₂CH₂-, -CH(CH₂CH₃)-, and -CH=CH-.

An "aryl" group is a substituent derived from an aromatic compound, including heterocyclic aromatic compounds having heteroatoms chosen from among nitrogen, oxygen and sulfur. An aryl group has a total of from 5 to 20 ring atoms, and has one or more rings which are separate or fused. Substitution on aryl groups of one or more halo, alkyl, alkenyl, hydroxy, alkoxy, alkanoyl or amido groups is permitted, with substitution by one or more halo groups being possible on alkyl, alkenyl, alkoxy, alkanoyl or amido groups.

An "aralkyl" group is an "alkyl" group substituted by an "aryl" group.

An "oxyl" substituent on a nitrogen atom is an -O substituent, i.e., the nitrogen and the oxyl form a nitroxyl group.

According to a preferred embodiment of the present invention X is O; in this case the lubricating composition contains at least an imidazolidone compound. Further it is preferred that R¹, R², R³ and R⁴ are independently hydrogen, alkyl, alkenyl, aryl or aralkyl, preferably all hydrogen. Also it is preferred that R⁵ and R⁶ are both hydrogen.

Further it is preferred that R¹ and R² combine to form a double bond with an O atom.

According to an especially preferred embodiment of the present invention, the lubricating composition contains at least one compound wherein X is O and at least one compound wherein X is S. In this case, the lubricant composition contains at least an imidazolidinethione compound and an imidazolidone compound. Preferably, the weight ratio of (imidazolidone compound):(imidazolidinethione compound) is from 1:10 to 1:1, preferably from 1:5 to 1:2.

Preferably, the one or more imidazolid(inethi)one compounds are present in an amount of from 0.05 to 20 wt.%, preferably from 0.1 to 10 wt.%, more preferably less than 5.0 wt.%, based on the total weight of the lubricating composition.

The imidazolid(inethi)one compounds as used in the present invention are either commercially available or can be prepared by various reactions that are known in the art. One recent example has been given in Jeon, H.S. et al. "Solid-phase synthesis of 2-imidazolidinethiones via Mitsunobu reaction of N-(2-hydroxyethyl)thioureas", Tetrahedron Letters, 48(3), 2007, pp. 439-441.

There are no particular limitations regarding the base oil used in lubricating composition according to the present invention, and various conventional mineral oils and synthetic oils may be conveniently used. For the purpose of this description, the term "base oil" is meant to also include a grease base stock.

The base oil used in the present invention may conveniently comprise mixtures of one or more mineral oils and/or one or more synthetic oils.

Mineral oils include liquid petroleum oils and solvent-treated or acid-treated mineral lubricating oil of the paraffinic, naphthenic, or mixed paraffinic/naphthenic type which may be further refined by hydrofinishing processes and/or dewaxing.

Suitable base oils for use in the lubricating oil composition of the present invention are Group I, Group II or Group III base oils, polyalphaolefins, Fischer-Tropsch derived base oils and mixtures thereof.

By "Group I" base oil, "Group II" base oil and "Group III" base oil in the present invention are meant lubricating oil base oils according to the definitions of American Petroleum Institute (API) categories I, II and III. Such API categories are defined in API Publication 1509, 15th Edition, Appendix E, April 2002.

Suitable Fischer-Tropsch derived base oils that may be conveniently used as the base oil in the lubricating oil composition of the present invention are those as for example disclosed in EP 0 776 959, EP 0 668 342, WO 97/21788, WO 00/15736, WO 00/14188, WO 00/14187, WO 00/14183, WO 00/14179, WO 00/08115, WO 99/41332, EP 1 029 029, WO 01/18156 and WO 01/57166.

Synthetic oils include hydrocarbon oils such as olefin oligomers (PAOs), dibasic acid esters, polyol esters, and dewaxed waxy raffinate. Synthetic hydrocarbon base oils sold by the Shell Group under the designation "XHVI" (trade mark) may be conveniently used.

The total amount of base oil incorporated in the lubricating composition of the present invention is preferably present in an amount in the range of from 60 to 92 wt.%, more preferably in an amount in the range of from 75 to 90 wt.% and most preferably in an amount in the range of from 75 to 88 wt.%, with respect to the total weight of the lubricating composition.

In a preferred embodiment of the present invention, the lubricating composition further comprises one or more additives such as anti-oxidants, anti-wear agents, dispersants, detergents, friction modifiers, viscosity index improvers, pour point depressants, corrosion inhibitors, demulsifiers, defoaming agents and seal fix or seal compatibility agents, etc.

As the person skilled in the art is familiar with the above and other additives, these are not further discussed here.

Said additives are typically present in an amount in the range of from 0.01 to 12.5 wt.%, based on the total weight of the lubricating composition, preferably in an amount in the range of from 0.05 to 10.0 wt.%, more preferably from 1.0 to 9.0 wt.% and most preferably in the range of from 2.0 to 5.0 wt.%, based on the total weight of the lubricating composition.

As the lubricating composition may also be in the form of a grease, the base oil as contained in the lubricating composition may contain or be compounded with one or more thickeners such as metallic soaps, organic substances or inorganic substances, for example, lithium soaps, lithium complex soaps, sodium terephthalate, urea/urethane compounds and clays.

Preferably, the lubricating composition has a kinematic viscosity in the range of from 2 to 80 mm²/s at 100 °C, more preferably in the range of from 3 to 70 mm²/s, most preferably in the range of from 4 to 50 mm²/s.

The lubricating compositions of the present invention may be conveniently prepared by admixing the one or more base oils and, optionally, one or more additives that are usually present in lubricating compositions, for example as herein before described, with mineral and/or synthetic base oil. Preferably, and as is customary in the art, the one or more imidazolid(inethi)one compounds have a sufficiently small particle size (e.g. below 50 µm, preferably below 20 µm) to allow easy dispersion thereof in the lubricating composition.

In another aspect the present invention provides a method of improving friction reduction properties, preferably in an internal combustion engine, which method comprises lubricating (preferably said internal combustion engine) with the lubricating composition according to the present invention.

Also the present invention provides the use of the lubricating composition according to the present invention in order to improve friction reduction properties.

The person skilled in the art will readily understand that the lubricating composition may also be suitably used for other uses than in an internal combustion engine, where friction reduction and anti-wear properties play a role, e.g., as automatic transmission fluid, turbine lubricant, gear lubricant, compressor lubricant, metal-working lubricant, hydraulic fluid, etc.

The present invention is described below with reference to the following Examples, which are not intended to limit the scope of the present invention in any way.

### Examples

### Lubricating Oil Compositions

Table 1 indicates the composition of the lubricating oil compositions that were tested; the amounts of the components are given in wt.%, based on the total weight of the fully formulated formulations.

The core lubricating composition as used in the formulations of Table 1 was a mixture of PAO 4 and PAO 5 base oils and ester base fluid further containing conventional anti-wear additives, anti-foam agent, detergents, anti-oxidant and viscosity modifier.

The core lubricating composition had a viscosity of about 43 mm²/s at 100 °C and a maximum viscosity of about 8 mm²/s at 40 °C.

The imidazolidinethione component as used in Examples 1 and 3 was 2-imidazolidinethione (i.e. formula (I) wherein X is S and R¹-R⁶ are all H) available from e.g. Fluka (under the trade designation "03940") or Sigma Aldrich.

The imidazolidone component as used in Examples 2 and 3 was 2-imidazolidone (i.e. formula (I) wherein X is O and R¹-R⁶ are all H) available from e.g. Fluka (under the trade designation "03850") or Sigma Aldrich.

The friction modifier as used in Comparative Example 2 was a conventional organic friction modifier available from e.g. Oleon NV (under the trade designation "Radiasurf 7149", containing 49% glycerol mono-oleate).

The compositions of Examples 1-3 were obtained by dissolving the imidazolidinethione and imidazolidone components with methanol, adding the obtained solution to the core lubricating composition which had previously been heated up to a temperature of 60 °C. The obtained compositions were stirred until the methanol had been evaporated (about 4 hours).

Comparative Example 2 was obtained by simply mixing the Radiasurf 7149 friction modifier with the core lubricating composition.

**Table 1**

| Component (wt.%) | Example 1 | Example 2 | Example 3 | Comp. Ex. 1 | Comp. Ex. 2 |
|---|---|---|---|---|---|
| Core lubricating composition | 98.5 | 99.5 | 98 | 100 | 98.5 |
| Imidazolidine thione | 1.5 | - | 1.5 | - | - |
| Imidazolidone | | 0.5 | 0.5 | - | - |
| Friction modifier | - | - | - | - | 1.5 |
| TOTAL | 100 | 100 | 100 | 100 | 100 |

### Friction Modification Test

In order to demonstrate the friction properties of the present invention, friction coefficient measurements were made on a mini traction machine (MTM), available from PCS Instruments, United Kingdom.

In the test, the friction properties of the compositions of Examples 1-3 and Comparative Example 1 and 2 (see Table 1) were tested using the above mini traction machine.

The test used a steel ball rolling and sliding on a coated disc. In the standard configuration the ball was loaded against the face of the disc and the ball and disc were driven independently to create a mixed rolling/sliding contact. The frictional force between the ball and disc was measured by a force transducer. Additional sensors measured the applied load, the lubricant temperature and (optionally) the electrical contact resistance between the specimens and the relative wear between them.

Before testing, the ball and disc were immersed in the lubricant compositions, which were heated to 100°C. Subsequently, friction coefficient measurements were performed by running Stribeck curves (friction vs. speed) under the following test conditions:
- Load: 71 N
- Maximum Hertzian pressure: 1.25 GPa
- Lubricant temperature: 125°C
- Ball radius: 0.95 cm
- Slide roll ratio: 100%.
The slide roll ratio (SRR) was defined as defined as the ratio of sliding speed (U_{ball}-U_{disc}) to entrainment speed (U_{ball}+U_{disc})/2.

The measured friction coefficients are indicated in Table 2 below. The data have been chosen at low and moderate speed (20 and 200 mm/s) as this is deemed representative for lubricating conditions as found in the valve train of a combustion engine.

**Table 2**

| | Friction coefficient @ 20 mm/s | Percent reduction | Friction coefficient @ 200 mm/s | Percent reduction |
|---|---|---|---|---|
| Example 1 | 0.059 | 49.6 | 0.0709 | 31.8 |
| Example 2 | 0.097 | 17.1 | 0.0657 | 36.8 |
| Example 3 | 0.062 | 47.0 | 0.0511 | 50.9 |
| Comp. Ex. 1 | 0.117 | - | 0.104 | - |
| Comp. Ex. 2 | 0.089 | 24.0 | 0.0569 | 45.2 |

### Discussion

As can be learned from Table 2, the friction coefficients for Examples 1-3 were significantly reduced when compared with Comparative Example 1, both at low and moderate speed.

Further, the composition of Example 1 performed better than the conventional friction modifier of Comparative Example 2 at low speed, but slightly worse at high speed.

However, the composition of Example 3, containing a combination of an imidazolidinethione and imidazolidone compound (in a weight ratio of 3:1), outperformed Comparative Example 2 at both low and moderate speed.

### Anti-wear Test

The anti-wear properties of lubricating compositions according to the present invention containing an imidazolidinethione or an imidazolidone compound were determined in a Four-Ball Wear test similar to ASTM D 4172, wherein the speed of the upper ball was set at 1470 rpm instead of 1200 rpm.

It was found that desirable anti-wear properties were obtained for the compositions according to the present invention, indicating that according to the present invention good friction reduction properties can be obtained without compromising for anti-wear properties.

## Claims

1. A lubricating composition comprising:
- a base oil; and
- one or more imidazolid(inethi)one compounds having formula (I): 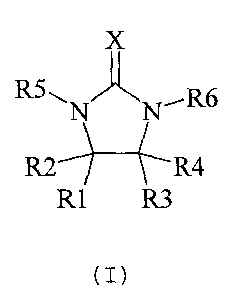 wherein X is O or S;
R¹, R² R³ and R⁴ are independently hydrogen, alkyl, alkenyl, aryl, aralkyl or NYR⁷; R⁷ is alkyl, alkenyl, aralkyl or R⁷ groups combine with N atoms to which they are attached and C atoms of an imidazolidinethione ring to form a five- to seven-membered heterocyclic ring; Y is hydrogen, alkyl, alkenyl, aryl, aralkyl, -CHR⁸-CHR⁹-COOR¹⁰, -CR¹¹R¹²NHR¹³ or -C(Z)NHR¹⁴ or oxyl; R⁸ and R⁹ are independently hydrogen or C₁-C₄ alkyl; R¹⁰, R¹¹ and R¹² are independently hydrogen, alkyl, alkenyl, aryl or aralkyl; R¹³ and R¹⁴ are independently alkyl, alkenyl, aralkyl or aryl; Z is O or S; provided that when X is S, one of R¹ and R² and one of R³ and R⁴ are not at the same time NYR⁷;
or R¹ and R³ combine with carbon atoms of an imidazolidinethione ring to form a C₅-C₈ cycloalkyl or cycloalkenyl ring;
or R² and R⁵ combine with carbon atoms of an imidazolidinethione ring to form a C₅-C₈ cycloalkyl or cycloalkenyl ring;
or R¹ and R² combine to form a double bond with an O or S atom;
R⁵ and R⁶ are independently hydrogen, alkyl, alkenyl, aryl, aralkyl, -CHR⁸-CHR⁹-COOR¹⁰, -CR¹¹R¹²NHR¹³ or -C(Z)NHR¹⁴.

2. Lubricating composition according to claim 1, wherein X is O.

3. Lubricating composition according to claim 1 or 2, wherein R¹, R², R³, R⁴, R⁵ and R⁶ are independently hydrogen, alkyl, alkenyl, aryl or aralkyl, preferably all hydrogen.

4. Lubricating composition according to any one of claims 1 to 3, wherein R⁵ and R⁶ are both hydrogen.

5. Lubricating composition according to any one of claims 1 to 4, wherein R¹ and R² combine to form a double bond with an O atom.

6. Lubricating composition according to any one of claims 1 to 5, containing at least one compound wherein X is O and at least one compound wherein X is S.

7. Lubricating composition according to claim 6, wherein the weight ratio of (the at least one compound wherein X is O):(the at least one compound wherein X is S) is from 1:10 to 1:1, preferably from 1:5 to 1:2.

8. Lubricating composition according to any one of claims 1 to 7, wherein the one or more imidazolid(inethi)one compounds are present in an amount of from 0.05 to 20 wt.%, based on the total weight of the lubricating composition.

9. A method of improving friction reduction properties, preferably in an internal combustion engine, which method comprises lubricating with a lubricating composition as described in any one of Claims 1 to 8.

10. Use of a lubricating composition according to any one of claims 1 to 8 in order to improve friction reduction properties, preferably in an internal combustion engine.
